# EUROPÄISCHE PATENTSCHRIFT

(11) **EP 3 814 477 B1**
(45) Veröffentlichungstag und Bekanntmachung des Hinweises auf die Patenterteilung: **08.04.2026**
(21) Anmeldenummer: 19755836.4
(22) Anmeldetag: 05.08.2019
(51) Int. Cl.: C12M 1/00, C12M 1/34

(54) **BIOPROZESSBEHÄLTER MIT OPTISCHER MESSVORRICHTUNG**
BIOPROCESS CONTAINER HAVING AN OPTICAL MEASURING DEVICE
RÉCIPIENTS DE BIOPROCÉDÉ MUNIS D'UN DISPOSITIF DE MESURE OPTIQUE

(30) Priorität: 31.10.2018 DE 102018008622
(43) Veröffentlichungstag der Anmeldung: 05.05.2021
(73) Patentinhaber: Sartorius Stedim Biotech GmbH, 37079 Göttingen (DE)
(72) Erfinder: HÖHSE, Marek, 37073 Göttingen (DE); GRIMM, Christian, 37308 Bad Heiligenstadt (DE); REGEN, Thomas, 37079 Göttingen (DE)
(74) Vertreter: Novagraaf International SA
(86) Internationale Anmeldenummer: PCT/EP2019/070961
(87) Internationale Veröffentlichungsnummer: WO 2020/088808

(56) Entgegenhaltungen:
- WO-A1-2008/016411
- DE-A1- 102010 007 559
- DE-B3- 102015 122 745
- DE-U1- 202009 010 255
- US-A1- 2012 244 609

## Beschreibung

Die vorliegende Erfindung betrifft einen Bioprozessbehälter mit einer optischen Messvorrichtung zur nicht-invasiven spektroskopischen Messung.

Aus dem Stand der Technik sind wiederverwendbare Bioprozessbehälter, wie beispielsweise Bioreaktoren oder Mischbehälter, bekannt. *Bioreaktoren* sind Behälter, in denen speziell herangezüchtete Mikroorganismen oder Zellen unter möglichst optimalen kontrollierten Bedingungen in einem Nährmedium kultiviert werden, um entweder die Zellen selbst, Teile von ihnen oder eines ihrer Stoffwechselprodukte zu gewinnen. Im Speziellen können in den Bioreaktoren feste (Biomasse), flüssige (Nährmedium) und/oder gasförmige (z.B. Luft, Sauerstoff, Kohlendioxid, Stickstoff) Phasen verarbeitet werden. In allen Bioprozessbehältern ist es hierbei jedoch erforderlich, optimale Bedingungen gewährleisten zu können. Hierzu werden üblicherweise verschiedene Parameter im Innenraum des Bioreaktors mit Hilfe von Sensoren gemessen bzw. überwacht, die in den Innenraum des Bioreaktors hineinragen. Mögliche zu messende Parameter sind beispielsweise der pH-Wert, der O₂-Wert und die Temperatur des in dem Bioprozessbehälter enthaltenen Mediums. Sollten Parameter von vordefinierten Optimalwerten abweichen, können mittels geeigneter Maßnahmen die Abweichungen korrigiert werden.

Eine Variante eines Sensors zur Überwachung des Mediums ist ein optischer Sensor bzw. eine optische Messvorrichtung, welcher bzw. welche in nichtinvasiver Weise mittels Spektroskopie bestimmte Parameter in dem Medium misst.

Bevor das Medium bzw. die einzelnen Bestandteile des Mediums in den Bioprozessbehälter aufgenommen werden können, ist es erforderlich, den Bioprozessbehälter inklusive der an dem Bioprozessbehälter montierten Sensoren, wie beispielsweise dem oben genannten optischen Sensor, zu sterilisieren. Dies kann beispielsweise durch Autoklavieren oder durch Dampfsterilisation erfolgen. Ein derartiger Sterilisationsprozess stellt jedoch eine hohe thermische Belastung des optischen Sensors dar, da bereits geringe thermische Verformungen Einfluss auf die Justierung der einzelnen Komponenten des optischen Sensors haben können. Verschiedene spektroskopische Techniken benötigen verschiedene exakte Anordnungen zur optimalen Spektrenakquisition. Gerade bei der Transmissionsmessung ist eine exakte Ausrichtung der Anregungsstrahlung und des Detektionskanals erforderlich.

Abweichungen, die durch Sterilisationsprozesse an dem optischen Sensor entstehen, werden häufig durch statistische Behandlungen der Spektren im Nachhinein korrigiert. Allerdings ist jede Korrektur fehlerbehaftet und birgt das Risiko, dass unerwünschte Abweichungen auch nach der Korrektur noch bestehen. Darüber hinaus können durch die Korrektur auch wichtige Informationen verloren gehen.

Zusätzlich müssen nach einer bestimmten Anzahl von Sterilisationsprozessen Dichtungen an dem optischen Sensor getauscht werden. Dieser Tausch erfordert das Einsenden des optischen Sensors zum Service und nach Austausch der Dichtungen einen erneuten Einbau und eine erneute Feinjustierung in den bzw. dem Bioprozessbehälter.

Aus der Druckschrift DE 20 2009 010 255 U1 ist ein Bioreaktor mit Fenster bekannt. Der Bioreaktor umfasst mindestens ein transparentes Fenster, dessen Innenseite von einem in einem Reaktorinnenraum anordenbaren Medium berührbar ist. Das Fenster weist auf seiner dem Reaktorinnenraum zugewandten Innenseite eine photokatalytische Beschichtung auf, die von der Innenseite abgewandten Außenseite her mit mindestens einer Lichtquelle aktivierbar ist.

Die Druckschrift WO 2008/016411 A1 offenbart einen Port zur Verwendung mit einem Bioreaktorgefäß, welcher umfasst: i) ein Basiselement, umfassend einen hohlen rohrförmigen Abschnitt und eine Grundplatte, die konfiguriert ist, um versiegelnd an einem Loch in der Wand des Bioreaktorgefäßes befestigt zu werden, ii) ein hohles, im Allgemeinen röhrenförmiges Buchsenelement zum Aufnehmen von elektrischen, optischen, mikrofluidischen und/oder chemischen Überwachungskomponenten, wobei das Buchsenelement in die Bohrung des rohrförmigen Abschnitts des Basiselements passt, wobei sowohl das Basiselement als auch das Buchsenelement den Zugang zum Inhalt des Bioreaktors ermöglichen; iii) eine in das Buchsenelement eingesetzte Überwachungsanordnung, deren Anordnung Mittel zum Bereitstellen eingehender optischer und / oder elektrischer Signale und Mittel zum Sammeln und Übertragen von Messsignalen umfasst, die aus der Wechselwirkung eingehender optischer und / oder elektrischer Signale mit dem Inhalt eines Bioreaktors resultieren; und iv) eine Abdeckung, die die Position und Ausrichtung der Komponenten ii) und iii) relativ zum Basiselement beibehält.

Zudem offenbart die Druckschrift DE 10 2015 122 745 B3 einen Behälter mit einem in seinen Behälterinnenraum hineinragenden Messzellengehäuse einer optischen Messzelle. Das Messzellengehäuse weist einen Messspalt auf, der von zwei einander in einem Abstand gegenüberliegenden Seitenflächen und einer die Seitenflächen verbindenden Verbindungsfläche begrenzt wird. Die Seitenflächen weisen jeweils ein optisches Fenster auf, wobei dem ersten Fenster mindestens eine erste optische Faser und dem zweiten Fenster mindestens eine zweite optische Faser vorgelagert werden kann. Das Messzellengehäuse weist den Fenstern vorgelagerte Aufnahmekanäle zur Aufnahme der jeweils mindestens einen optischen Faser auf. Die Aufnahmekanäle sind nachträglich von außen her mit den optischen Fasern bestückbar. Das Messzellengehäuse mit den Fenstern und Aufnahmekanälen ist fest mit der Wandung des Behälterinnenraumes verbunden.

Der vorliegenden Erfindung liegt somit die Aufgabe zugrunde, einen Bioprozessbehälter mit einer optischen Messvorrichtung zur nicht-invasiven spektroskopischen Messung bereitzustellen, die es ermöglicht, thermische Einwirkungen durch einen Sterilisationsprozess auf die optische Messvorrichtung zu verhindern.

Diese Aufgabe wird durch einen Bioprozessbehälter mit einer optischen Messvorrichtung zur nicht-invasiven spektroskopischen Messung gelöst, welcher umfasst:
- ein Behältergehäuse, welches geeignet ist, zumindest ein zu messendes Fluid aufzunehmen;
- ein Portgehäuse, welches mit dem Behältergehäuse verbunden ist und
- gegenüber dem Innenraum des Behältergehäuses abgeschlossen ist;
- zumindest ein Strahlung emittierendes Element, welches dazu ausgelegt ist, elektromagnetische Strahlung durch das zumindest eine in dem Behältergehäuse enthaltenen Fluid zu senden;
- zumindest ein Strahlung empfangendes Element, welches dazu ausgelegt ist, die Strahlung, welche von dem Strahlung emittierenden Element ausgesendet wurde, zumindest teilweise zu empfangen; und
- zumindest einen Messeinsatz, welcher das zumindest eine Strahlung emittierende Element und das zumindest eine Strahlung empfangende Element hält bzw. stützt,

wobei der Messeinsatz in das Portgehäuse zumindest teilweise einschiebbar ist und im eingeschobenen Zustand mit dem Portgehäuse lösbar verbunden ist;
wobei der Messeinsatz Halteaussparungen aufweist, in die das Strahlung emittierende Element und das Strahlung empfangende Element einsetzbar sind, und
wobei der Messeinsatz umfasst:
   - zumindest eine Haltefläche, in der die Halteaussparungen ausgebildet sind und welche im in das Portgehäuse eingesetzten Zustand einem Fenster, einem ersten Umlenkelement und/oder zweiten Umlenkelement gegenüber angeordnet ist, und
   - zumindest eine Verbindungsfläche, welche mit der zumindest einen Haltefläche verbunden ist und dazu ausgelegt ist, mit dem Portgehäuse im eingesetzten Zustand verbunden zu sein.

Unter einer *"nicht-invasiven"* Messmethode wird ein Messverfahren verstanden, das ein Messen von außerhalb des Bioprozessbehälters ermöglicht. Es ist nicht erforderlich, dass Messgeräte der Messvorrichtung in den Innenraum des Bioprozessbehälters eindringen. Der Innenraum des Portgehäuses, in dem die gegenüber der Sterilisation empfindlichen Komponenten der optischen Messvorrichtung enthalten sind, steht nicht in Verbindung mit dem Innenraum des Bioprozessbehälters.

Der zumindest eine Messeinsatz, welcher das zumindest eine Strahlung emittierende Element und das zumindest eine Strahlung empfangende Element hält bzw. stützt, kann zusammen mit diesen Elementen in einfacher Weise in das Portgehäuse eingeschoben bzw. aus dem Portgehäuse entfernt werden. Somit sind die gegenüber der Sterilisation empfindlichen Elemente während des Sterilisationsprozesses von dem Bioprozessbehälter entfernt. Erst nachdem der Sterilisationsprozess abgeschlossen ist, wird der Messeinsatz zusammen mit dem zumindest eine Strahlung emittierenden Element und dem zumindest eine Strahlung empfangende Element in das Portgehäuse eingeschoben. Ist der Messeinsatz ordnungsgemäß in dem Portgehäuse eingesetzt, sind auch das eine Strahlung emittierende Element und das zumindest eine Strahlung empfangende Element ordnungsgemäß eingesetzt, so dass eine
exakte spektroskopische Messung erfolgen kann. Insbesondere sind durch den Messeinsatz das eine Strahlung emittierende Element und das zumindest eine Strahlung empfangende Element immer exakt zueinander ausgerichtet. Eine statistische Korrektur der Spektrenauswertung ist somit nicht mehr notwendig.

Die Halteaussparungen für das Strahlung emittierende Element und das Strahlung empfangende Element ermöglichen es, dass sich, sobald der Messeinsatz in das Portgehäuse eingesetzt ist, die genannten Elemente immer in einer optimalen Position bzw. Ausrichtung befinden. Insbesondere können durch den Messeinsatz mit Halteaussparungen das Strahlung emittierende Element und das Strahlung empfangende Element zusammen bzw. gleichzeitig in das Portgehäuse eingesetzt werden. Dies hat eine Zeit- und Kostenersparnis zur Folge.

Als *"eingesetzter Zustand"* wird der Zustand verstanden, in dem der Messeinsatz vollständig in das Portgehäuse eingesetzt ist bzw. eine derartige Position in dem Portgehäuse eingenommen hat, dass der optische Messeinsatz zur spektroskopischen Messung bereit ist.

Die zumindest eine Haltefläche ist vorzugsweise so ausgerichtet, dass sie parallel gegenüber einem Fenster, einem ersten Umlenkelement und/oder zweiten Umlenkelement angeordnet ist.

Die zumindest eine Verbindungsfläche ist mit der zumindest einen Haltefläche verbunden und ist vorzugsweise derart ausgerichtet, dass diese mit einer Innenfläche des Portgehäuses zumindest bereichsweise im eingesetzten Zustand des Messeinsatzes in Verbindung kommt, um mit dem Portgehäuse lösbar verbunden zu sein bzw. zu werden.

Vorzugsweise umfasst das Portgehäuse zumindest ein Fenster, welches ausgelegt ist, elektromagnetische Strahlung zwischen dem Innenraum des Behältergehäuses und einem Innenraum des Portgehäuses zumindest teilweise passieren zu lassen.

Das Fenster stellt vorzugsweise eine Fensterfläche dar, welche ein Teil des Portgehäuses ist. Das Fenster kann dabei aus einem Material ausgebildet sein, das dazu ausgelegt ist, elektromagnetische Strahlung zumindest teilweise passieren zu lassen. Dies kann über das gesamte elektromagnetische Spektrum oder nur über einen bestimmten Bereich des elektromagnetischen Spektrums erfolgen. Die Auswahl des Fensters hängt somit von der gewünschten Messung ab bzw. von dem gewünschten auszuwertenden Spektrenbereich.

Es kann ein Fenster vorgesehen sein, hinter dem alle Strahlung emittierenden Elemente und Strahlung empfangenden Elemente in dem Innenraum des Portgehäuses angeordnet sind. Es ist jedoch auch möglich, dass ein Fenster nur vor einzelnen Strahlung emittierenden Elementen und/oder Strahlung empfangenden Elementen angeordnet ist.

Das Fenster ermöglicht es, dass zwischen dem Innenraum des Bioprozessbehälters und dem Innenraum des Portgehäuses eine optische Verbindung hergestellt werden kann, um eine spektroskopische Messung an dem in dem Bioprozessbehälter enthaltenen Medium vornehmen zu können. Es muss jedoch keine Fluidverbindung zwischen dem Innenraum des Bioprozessbehälters und dem Innenraum des Portgehäuses hergestellt werden.

Ferner ist es bevorzugt, dass das Portgehäuse zumindest einen Messspalt bzw. eine Messvertiefung aufweist, in den bzw. in die das zu messende Fluid aus dem Innenraum des Behältergehäuses einströmbar ist.

Ein Innenraum des Messspalts stellt einen Teil des Innenraums des Bioprozessgehäuses dar, in den ein Teil des in dem Bioprozessgehäuse enthaltenen Mediums einströmen kann. Das in dem Messspalt enthaltene Medium kann mittels Spektroskopie von der optischen Messvorrichtung gemessen werden und somit Rückschlüsse auf den gesamten Inhalt des Bioprozessgehäuses gezogen werden.

Vorzugsweise weist der Messspalt zumindest zwei sich gegenüberliegende Fenster auf, die derart voneinander beabstandet sind, dass zwischen den Fenstern der Messspalt ausgebildet ist.

Mit anderen Worten sind zwei sich gegenüberliegende Flächen des Portgehäuses, die den Messspalt ausbilden, zumindest bereichsweise als Fenster, wie bereits oben beschrieben, ausgebildet. Die zwei gegenüberliegenden Fenster ermöglichen dabei, dass elektromagnetische Strahlung im Rahmen der spektroskopischen Messung durch den Messspalt hindurchgesendet werden kann.

In bevorzugter Weise ist der Messspalt durch eine Strahlungsumlenkungsvorrichtung ausgebildet,
wobei die Strahlungsumlenkungsvorrichtung zumindest ein erstes Umlenkungselement und zumindest ein zweites Umlenkungselement aufweist,
wobei das erste und zweite Umlenkungselement einander gegenüber angeordnet sind und zumindest bereichsweise voneinander beabstandet sind, so dass der Messspalt zwischen dem ersten und zweiten Umlenkungselement ausgebildet ist.

Alternativ kann eine Strahlungsumlenkungsvorrichtung mit dem Messeinsatz verbunden sein und zusammen mit dem Messeinsatz in das Portgehäuse einschiebbar sein,
wobei die Strahlungsumlenkungsvorrichtung zumindest ein erstes Umlenkungselement und zumindest ein zweites Umlenkungselement aufweist,
wobei das erste und zweite Umlenkungselement derart angeordnet sind, dass diese durch die zumindest zwei Fenster und den Messspalt beabstandet sind.

Im Gegensatz zu der oben beschriebenen Ausführungsform, in der der Messspalt durch das erste und zweite Umlenkungselement definiert wird, wird in dieser Ausführungsform der Messspalt durch zumindest zwei gegenüberliegende Fenster ausgebildet. Eigenschaften eines solchen Fensters wurden bereits oben beschrieben. Das Portgehäuse ist somit bevorzugt so ausgebildet, dass das erste und zweite Umlenkungselement im Innenraum des Portgehäuses anordenbar sind und vorzugsweise mit dem Messeinsatz verbunden sind. Hierdurch können das erste und zweite Umlenkungselement ebenfalls mit dem Messeinsatz aus dem Portgehäuse entfernt und wieder eingesetzt werden.

Vorzugsweise ist das erste Umlenkungselement ausgelegt ist, elektromagnetische Strahlung, welche von dem Strahlung emittierenden Element ausgesendet wird, zu empfangen und anschließend an die zweite Umlenkungseinheit derart umzulenken, dass die Strahlung den Messspalt passiert, und
wobei die zweite Umlenkungseinheit ausgelegt ist, die Strahlung von der ersten Umlenkungseinheit zu empfangen und im Anschluss an das Strahlung empfangende Element umzulenken.

Das erste Umlenkungselement empfängt die elektromagnetische Strahlung von dem Strahlung emittierenden Element aus einer ersten Richtung und lenkt die Strahlung in eine zweite Richtung um, so dass die Strahlung den Messspalt passiert. Die erste Richtung ist dabei von der zweiten Richtung verschieden. Das zweite Umlenkungselement empfängt zumindest einen Teil der Strahlung von dem ersten Umlenkungselement und lenkt die Strahlung derart um, dass die Strahlung wieder in der ersten Richtung zu dem Strahlung empfangenden Element gesendet wird. Optional kann eine Lochblende in den Strahlengang eingebracht werden. Mit Hilfe einer derartigen Konfiguration des Strahlung emittierenden Elements und des Strahlung empfangenden Elements kann somit eine Transmissionsmessung erfolgen.

Das erste und zweite Umlenkungselement ist dabei aus einem Material gefertigt, das durchlässig für elektromagnetische Strahlung ist; entweder über das gesamte elektromagnetische Spektrum oder nur für einen oder mehrere bestimmte Spektrenbereiche.

Das erste und zweite Umlenkungselement stellen keine kritischen Elemente dar, die während eine Sterilisationsprozesses der thermischen Belastung ausgesetzt sein können, ohne mögliche Fehler im späteren Messergebnis zur Folge zu haben. Diese können somit während des Sterilisationsprozesses an dem Bioprozessbehälter verbleiben.

In einer bevorzugten Ausführungsform ist das erste Umlenkungselement ausgelegt, elektromagnetische Strahlung, welche von dem Strahlung emittierenden Element ausgesendet wird, zu empfangen und anschließend zu dem Messspalt umzulenken, und
wobei ein Strahlung empfangendes Element ist unterhalb des Messspalts angeordnet ist.

Das Strahlung empfangende Element unterhalb des Messspalts ist dabei ausgelegt, den Anteil der elektromagnetischen Strahlung zu messen, der nach Reaktion bzw. in Kontakt treten mit dem Medium in dem Messspalt zu dem Strahlung empfangende Element abgestrahlt wird. Vorzugsweise handelt es sich hierbei um eine 90°-Detektion.

Eine derartige Konfiguration kann eine eigene Ausführungsform sein, bei der dann das zweite Umlenkungselement ohne Funktion ist. Das zweite Umlenkungselement begrenzt hier lediglich den Messspalt.

Eine derartige Konfiguration kann jedoch auch zusätzlich zu der bereits oben beschriebenen Transmissionsmessung erfolgen. Das heißt, dass hier mehrere Strahlung empfangende Elemente vorhanden sind.

Vorzugsweise sind das Strahlung emittierende Element und das Strahlung empfangende Element an derselben Position unterhalb des Messspalts angeordnet.

Mit Hilfe einer derartigen Anordnung des Strahlung emittierenden Elements sowie des Strahlung empfangenden Elements ist eine Reflexionsmessung (z.B. Raman-Spektroskopie, Fluoreszenzspektroskopie, UV-Vis-Spektroskopie oder Nahinfrarotspektroskopie) möglich. Mit anderen Worten wird eine elektromagnetische Strahlung von dem Strahlung emittierenden Element in Richtung zu dem Messspalt ausgesendet und dort von dem in dem Messspalt enthaltenen Medium reflektiert. Das Strahlung empfangende Element kann diesen reflektierten Anteil messen, wobei sich das Strahlung empfangende Element an derselben Position befindet wie das Strahlung emittierende Element. Vorzugsweise sind das Strahlung emittierende Element und das Strahlung empfangende Element als eine Einheit ausgebildet.

Alternativ ist es auch möglich, dass sich das Strahlung emittierende Element und das Strahlung empfangende Element an derselben Position unterhalb des ersten Umlenkungselements befindet, um den von dem Medium reflektierten Anteil zu messen.

Weiterhin kann eine Fläche des zweiten Umlenkungselements, welche dem Messspalt zugewandt ist, zumindest bereichsweise diffus streuend ausgebildet sein,
wobei das erste Umlenkungselement ausgelegt ist, elektromagnetische Strahlung, welche von dem Strahlung emittierenden Element ausgesendet wird, zu empfangen und anschließend zu dem Messspalt und/oder der diffus streuenden Fläche des zweiten Umlenkungselements umzulenken, und
das Strahlung empfangende Element kann derart angeordnet sein, dass dieses die elektromagnetische Strahlung, welche von dem zu messenden Fluid und/oder der diffus streuenden Fläche reflektiert wird, messen kann.

Mit Hilfe dieser Ausführungsform sind eine Transflexionsmessung (Reflexion der elektromagnetischen Strahlung an der diffus streuenden Fläche) und/oder eine Reflexionsmessung (Reflexion der elektromagnetischen Strahlung an dem Medium) möglich.

Vorzugsweise befindet sich das Strahlung emittierende Element und das Strahlung empfangende Element unterhalb des ersten Umlenkungselements. Ähnlich zu der oben beschriebenen Reflexionsmessung können die beiden Elemente auch hier als Einheit ausgebildet sein. Insbesondere sind das Strahlung emittierende Element und das Strahlung empfangende Element vorzugsweise an derselben Position angeordnet.

Vorzugsweise ist das erste und zweite Umlenkungselement jeweils ein Prisma oder weist jeweils eine Strahlung reflektierende Fläche auf.

Weist das erste und zweite Umlenkungselement eine Strahlung reflektierende Fläche auf, ist eine Fläche des ersten und zweiten Umlenkungselements zumindest bereichsweise mit einem reflektierenden Material, wie beispielsweise Gold, beschichtet. Die Fläche ist dabei so ausgerichtet, dass die elektromagnetische Strahlung von der ersten zur zweiten Richtung und umgekehrt umlenkbar ist. Mit anderen Worten bildet die reflektierende Fläche einen Umlenkspiegel.

Weiterhin ist es bevorzugt, dass das erste und zweite Umlenkungselement auf zumindest einem Fenster angeordnet ist.

Vorzugsweise ist das zumindest eine Fenster dabei nicht vollständig durch das erste und zweite Umlenkungselement überdeckt. Beispielsweise ist eine Bodenfläche des Messspalts nicht durch das erste und zweite Umlenkungselement überdeckt. In diesem Fall können mehrere Messverfahren innerhalb einer optischen Messvorrichtung kombiniert werden (siehe beispielsweise die oben beschriebene Reflexionsmessung oder Transflexionsmessung).

Im Speziellen könnte ein erstes Strahlung emittierendes Element und ein erstes Strahlung empfangendes Element mit Hilfe des Messeinsatzes derart positioniert werden, dass über das erste und zweite Umlenkungselement eine Transmissionsmessung erfolgen könnte. Gleichzeitig könnte jedoch ein zweites Strahlung emittierendes Element und ein zweites Strahlung empfangendes Element mit Hilfe des Messeinsatzes unterhalb der Bodenfläche des Messeinsatzes (Fläche nur mit Fenster) derart positioniert werden, dass parallel eine Reflexionsmessung (z.B. Raman-Spektroskopie, Fluoreszenzspektroskopie, UV-Vis-Spektroskopie oder Nahinfrarotspektroskopie) über die Bodenfläche des Messspalts erfolgen kann.

Vorzugsweise ist der Messeinsatz in das Portgehäuse einklemmbar, mit dem Portgehäuse verrastbar und/oder verschraubbar.

Durch derart lösbare Verbindungen zwischen dem Messeinsatz und dem Portgehäuse kann der Messeinsatz in einfacher Weise mit dem Portgehäuse verbunden werden und vor einem Sterilisationsprozess auch wieder in einfacher Weise gelöst und entfernt werden. Darüber hinaus ist der Messeinsatz durch die oben genannten Verbindungsarten sicher mit dem Portgehäuse verbunden.

Vorzugsweise ist der Bioprozessbehälter ein Bioreaktor.

Als wiederverwendbarer Bioreaktor kann der Bioreaktor zum Beispiel aus Glas oder Stahl gefertigt sein. Es ist jedoch auch denkbar, dass der Bioprozessbehälter ein Mischbehälter ist, in dem keine Kultivierung stattfindet, sondern zumindest eine Fluid gemischt wird, wo es jedoch notwendig ist, die einzelnen Zustandsparameter des Fluids zu überwachen. Alternativ kann es sich um ein Lagerbehältnis für zumindest ein Fluid handeln. Sowohl der Mischbehälter als auch das Lagerbehältnis sind vorzugsweise aus Glas oder Stahl gefertigt.

Vorzugsweise ragt das Portgehäuse zumindest teilweise in den Innenraum des Behältergehäuses.

Gemäß einem weiteren Aspekt der vorliegenden Erfindung wird die vorliegende technische Aufgabe durch eine optische Messvorrichtung zur nicht-invasiven spektroskopischen Messung für einen Bioprozessbehälter gelöst, welche umfasst:
- ein Portgehäuse, welches mit einem Behältergehäuse des Bioprozessbehälters verbindbar ist und derart ausgebildet ist, dass dieses im verbundenen Zustand gegenüber dem Innenraum des Behältergehäuses abgeschlossen ist;
- zumindest ein Strahlung emittierendes Element, welches dazu ausgelegt ist, elektromagnetische Strahlung durch zumindest ein in dem Behältergehäuse enthaltenes Fluid zu senden;
- zumindest ein Strahlung empfangendes Element, welches dazu ausgelegt ist, die Strahlung, welche von dem Strahlung emittierenden Element ausgesendet wurde, zumindest teilweise zu empfangen; und
- zumindest einen Messeinsatz, welcher das zumindest eine Strahlung emittierende Element und das zumindest eine Strahlung empfangende Element hält bzw. stützt,

wobei der Messeinsatz in das Portgehäuse zumindest teilweise einschiebbar ist und im eingeschobenen Zustand mit dem Portgehäuse lösbar verbunden ist;
wobei der Messeinsatz Halteaussparungen aufweist, in die das Strahlung emittierende Element und das Strahlung empfangende Element einsetzbar sind, und
wobei der Messeinsatz umfasst:
   - zumindest eine Haltefläche, in der die Halteaussparungen ausgebildet sind und welche im in das Portgehäuse eingesetzten Zustand einem Fenster, einem ersten Umlenkelement und/oder zweiten Umlenkelement gegenüber anordenbar ist, und
   - zumindest eine Verbindungsfläche, welche mit der zumindest einen Haltefläche verbunden ist und dazu ausgelegt ist, mit dem Portgehäuse im eingesetzten Zustand verbunden zu sein.

Vorzugsweise umfasst das Portgehäuse zumindest ein Fenster, welches ausgelegt ist, elektromagnetische Strahlung zwischen dem Innenraum des Behältergehäuses und einem Innenraum des Portgehäuses zumindest teilweise passieren zu lassen.

Ferner ist es bevorzugt, dass das Portgehäuse zumindest einen Messspalt bzw. eine Messvertiefung aufweist, in den bzw. in die das zu messende Fluid aus dem Innenraum des Behältergehäuses einströmbar ist.

Vorzugsweise weist der Messspalt zumindest zwei sich gegenüberliegende Fenster auf, die derart voneinander beabstandet sind, dass zwischen den Fenstern der Messspalt ausgebildet ist.

Ferner ist es bevorzugt, dass der Messspalt durch eine Strahlungsumlenkungsvorrichtung ausgebildet ist,
wobei die Strahlungsumlenkungsvorrichtung zumindest ein erstes Umlenkungselement und zumindest ein zweites Umlenkungselement aufweist,
wobei das erste und zweite Umlenkungselement einander gegenüber angeordnet sind und zumindest bereichsweise voneinander beabstandet sind, so dass der Messspalt zwischen dem ersten und zweiten Umlenkungselement ausgebildet ist.

Vorzugsweise ist das erste Umlenkungselement ausgelegt ist, elektromagnetische Strahlung, welche von dem Strahlung emittierenden Element ausgesendet wird, zu empfangen und anschließend an die zweite Umlenkungseinheit derart umzulenken, dass die Strahlung den Messspalt passiert, und
wobei die zweite Umlenkungseinheit ausgelegt ist, die Strahlung von der ersten Umlenkungseinheit zu empfangen und im Anschluss an das Strahlung empfangende Element umzulenken.

Ferner ist es bevorzugt, dass das erste Umlenkungselement ausgelegt ist, elektromagnetische Strahlung, welche von dem Strahlung emittierenden Element ausgesendet wird, zu empfangen und anschließend zu dem Messspalt umzulenken, und
dass ein Strahlung empfangendes Element unterhalb des Messspalts angeordnet ist.

Vorzugsweise sind das Strahlung emittierende Element und das Strahlung empfangende Element an derselben Position unterhalb des Messspalts angeordnet.

Vorzugsweise ist eine Fläche des zweiten Umlenkungselements, welche dem Messspalt zugewandt ist, zumindest bereichsweise diffus streuend ausgebildet ist,
wobei das erste Umlenkungselement ausgelegt ist, elektromagnetische Strahlung, welche von dem Strahlung emittierenden Element ausgesendet wird, zu empfangen und anschließend zu dem Messspalt und/oder der diffus streuenden Fläche des zweiten Umlenkungselements umzulenken, und
wobei das Strahlung empfangende Element derart angeordnet ist, dass dieses die elektromagnetische Strahlung, welche von dem zu messenden Fluid und/oder der diffus streuenden Fläche reflektiert wird, messen kann

Vorzugsweise weist das erste und zweite Umlenkungselement jeweils ein Prisma ist oder jeweils eine Strahlung reflektierende Fläche auf.

Es ist bevorzugt, dass das erste und zweite Umlenkungselement auf zumindest einem Fenster angeordnet sind.

Ferner ist es bevorzugt, dass der Messeinsatz in das Portgehäuse einklemmbar, mit dem Portgehäuse verrastbar und/oder verschraubbar ist.

Vorzugsweise ragt das Portgehäuse im verbundenen Zustand zumindest teilweise in den Innenraum des Behältergehäuses.

Diese und andere Ziele, Merkmale und Vorteile der vorliegenden Erfindung werden durch das Studium der folgenden detaillierten Beschreibung bevorzugter Ausführungsformen und der beigefügten Zeichnungen klarer. Weiterhin wird darauf hingewiesen, dass, obwohl Ausführungsformen separate beschrieben werden, einzelne Merkmale dieser Ausführungsformen zu zusätzlichen Ausführungsformen kombiniert werden können.
- Figur 1a): zeigt eine Draufsicht auf einen Teilbereich eines Behältergehäuses eines Bioprozessbehälters mit einer optischen Messvorrichtung gemäß einer ersten Ausführungsform;
- Figur 1b): zeigt eine Schnittansicht entlang der Schnittachse B-B in Figur 1a);
- Figur 2a): zeigt eine Draufsicht auf einen Teilbereich eines Behältergehäuses eines Bioprozessbehälters mit einer optischen Messvorrichtung gemäß einer zweiten Ausführungsform;
- Figur 2b): zeigt eine Schnittansicht entlang der Schnittachse A-A in Figur 2a);
- Figur 3a): zeigt eine Draufsicht auf einen Teilbereich eines Behältergehäuses eines Bioprozessbehälters mit einer optischen Messvorrichtung gemäß einer dritten Ausführungsform;
- Figur 3b): zeigt eine Schnittansicht entlang der Schnittachse B-B in Figur 3a).
- Figur 4: zeigt Figur 1b) mit eingezeichnetem Verlauf der elektromagnetischen Strahlung;
- Figur 5: zeigt Figur 2b) mit eingezeichnetem Verlauf der elektromagnetischen Strahlung;
- Figur 6: zeigt Figur 3b) mit eingezeichnetem Verlauf der elektromagnetischen Strahlung;
- Figur 7: zeigt eine Schnittansicht durch einen Teil eines Bioprozessbehälters mit einer optischen Messvorrichtung mit einer 90°-Detektion; und
- Figur 8: zeigt eine Schnittansicht durch einen Teil eines Bioprozessbehälters mit einer optischen Messvorrichtung mit einer Transflexionsmessung.

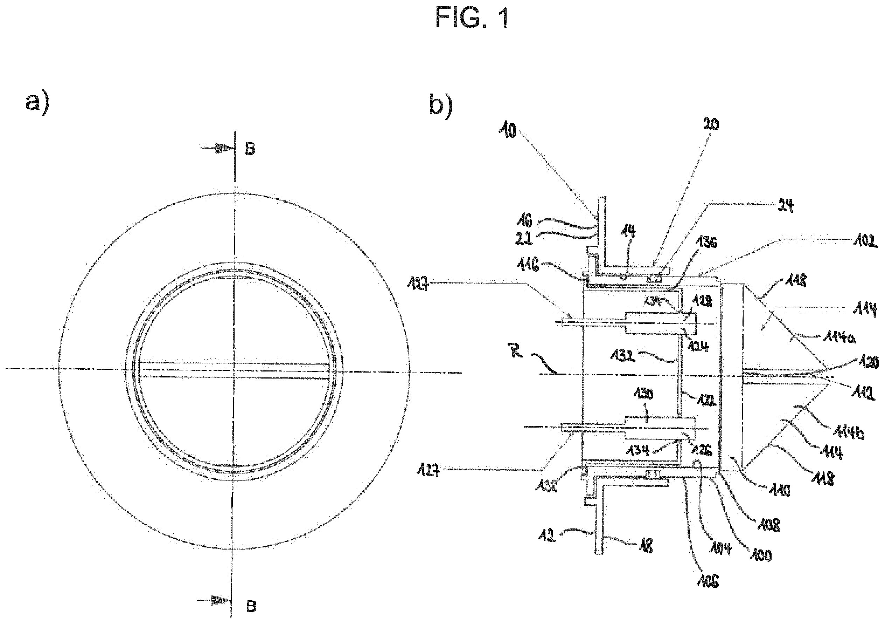

**Figuren 1a) und 1b****)** zeigen eine erste Ausführungsform einer optischen Messvorrichtung 100, die in einen Bioprozessbehälter 10 eingesetzt ist. Der Bioprozessbehälter 10 ist vorzugsweise ein Bioreaktor, ein Mischbehälter oder ein Lagerungstank aus vorzugsweise Stahl oder Glas. Der Bioprozessbehälter 10 ist dazu ausgelegt, zumindest ein Fluid aufzunehmen, das durch die optische Messvorrichtung 100 gemessen bzw. überwacht werden soll.

Um die optische Messvorrichtung 100 in den Bioprozessbehälter 10 einzusetzen bzw. mit dem Bioprozessbehälter 10 zu verbinden, weist ein Behältergehäuse 12 einen Behältergehäuseausschnitt 14 auf. Die Form und Größe des Behältergehäuseausschnitts 14 ist derart ausgelegt, dass die optische Messvorrichtung 100 von einer Außenseite 16 des Behältergehäuses 12 in Richtung zu einem Innenraum 18 des Behältergehäuses 12 einsetzbar ist. Vorzugsweise ist der Behältergehäuseausschnitt 14 kreisförmig. Insbesondere ist es bevorzugt, dass die optische Messvorrichtung 10 derart in den Bioprozessbehälter 10 einsetzbar ist, dass die optische Messvorrichtung 10 zumindest teilweise in den Innenraum 18 des Bioprozessbehälters 10 hineinragt.

Die optische Messvorrichtung 100 umfasst ein Portgehäuse 102, welches optische Messinstrumente aufnehmen kann und dessen Innenraum 104 derart ausgelegt ist, dass dieser fluiddicht gegenüber dem Innenraum 18 des Behältergehäuses 12 ist. Das Behältergehäuse 12 weist vorzugsweise einen Behältergehäusevorsprung 20 auf, der sich von einer Mantelfläche 22 des Behältergehäuses 12 entlang des Behältergehäuseausschnitts 14 in Richtung zu dem Innenraum 18 des Behältergehäuses 12 vorspringt. Vorzugsweise ragt der Behältergehäusevorsprung 20 senkrecht von der Mantelfläche 22 des Behältergehäuses 12 vor. Das Portgehäuse 102 wiederum weist ebenfalls eine Mantelfläche 106 auf und überlappt im eingesetzten Zustand zumindest teilweise mit dem Behältergehäusevorsprung 20. Wie in Figur 1b) gezeigt, welche eine Schnittansicht entlang der Schnittachse B-B aus Figur 1a) zeigt, kann die Mantelfläche 106 des Portgehäuses 102 über den Behältergehäusevorsprung 20 in den Innenraum 18 des Behältergehäuses 12 vorspringen. Um zu gewährleisten, dass der Bioprozessbehälter 10 fluiddicht ist und kein Fluid über den Behältergehäuseausschnitt 14 austreten kann, ist es vorteilhaft, zumindest einen Dichtungsring 24 zwischen dem Behältergehäusevorsprung 20 und der Mantelfläche 106 des Portgehäuses 102 anzuordnen.

An einem vorderen Ende 108 des Portgehäuses 102, welches in den Innenraum 104 des Portgehäuses 102 ragt, kann zumindest bereichsweise zumindest ein Fenster 110 ausgebildet sein. Das zumindest eine Fenster 110 kann aus Glas oder Kunststoff ausgebildet sein und transparent für elektromagnetische Strahlung über das gesamte Spektrum oder nur über einzelne oder einen Spektralbereich(e) sein. Das Fenster 110 erlaubt, dass optische Messinstrumente einen nicht-invasiven Zugang zu dem Innenraum 18 des Behältergehäuses 12 haben, der Innenraum 104 des Portgehäuses 102 jedoch fluiddicht gegenüber dem Innenraum 18 des Behältergehäuses 12 ist.

Wie in Figur 1b) gezeigt, erstreckt sich das Fenster 110 über das gesamte vordere Ende 108 des Portgehäuses 102.

Um eine Transmissionsmessung durchführen zu können, weist die optische Messvorrichtung 100 vorzugsweise einen Messspalt 112 auf. Der Messspalt 112 wird vorzugsweise durch zwei Prismen 114 (Umlenkungselemente) gebildet. Die Prismen 114 werden auf dem Fenster 110 angeordnet und sind derart voneinander beabstandet, dass zwischen den Prismen 114 der gewünschte Messspalt 112 ausgebildet ist. Der Messspalt 112 ist in Fluidverbindung mit dem Innenraum 18 des Behältergehäuses 12, so dass eine Teilmenge des Fluids aus dem Behältergehäuse 12 in den Messspalt 112 strömen kann und dort als Referenzmenge für den übrigen Inhalt des Behältergehäuses 12 gemessen werden kann. Wie in Figur 1b) gezeigt, weisen die Prismen 114 in einer Schnittansicht vorzugsweise eine Dreiecksform auf. Vorzugsweise handelt es sich um ein rechtwinkeliges Dreieck. Die Fläche 118 des Prismas 114, welche der Fläche gegenüberliegt, die den Messspalt 112 eingrenzt, ist dabei schräg gegenüber einer Rotationsachse R des Portgehäuses 102 angeordnet.

Alternativ kann der Messspalt 112 auch durch transparente Elemente (hier nicht gezeigt) ausgebildet sein, die eine ähnliche Form zu den Prismen 114 aufweisen. Die transparenten Elemente können Materialeigenschaften wie das Fenster 110 aufweisen. Im Gegensatz zu den Prismen 114 weisen die transparenten Elemente jedoch auf der schrägen Fläche 118 (schräg gegenüber der Rotationsachse R des Portgehäuses 102) zumindest bereichsweise eine reflektierende Beschichtung auf.

Obwohl Figur 1b) ein Fenster 110 zeigt, das das Portgehäuse 102 am vorderen Ende 108 verschließt, können die Prismen 114 oder die transparenten Elemente auch direkt das vordere Ende 108 des Portgehäuses 102 verschließen. Eine Bodenfläche 120 des Messspalts 112 kann dabei dann als Fenster 110 ausgebildet sein oder aber nicht transparent, ähnlich zu der Mantelfläche 104 des Portgehäuses 102 ausgebildet sein.

Ein hinteres Ende 116 des Portgehäuses 102 ist vorzugsweise offen zur Außenseite des Portgehäuses 102. Über dieses offene hintere Ende 116 ist zumindest ein Messeinsatz 122 zumindest teilweise in den Innenraum 104 des Portgehäuses 102 einschiebbar. Figur 1b) zeigt den Messeinsatz 122 im eingesetzten Zustand. Der Messeinsatz 122 ist lösbar mit dem Portgehäuse 102 verbunden, so dass der Messeinsatz 122 in einfacher Weise während eines Sterilisationsverfahrens aus dem Portgehäuse 102 entnommen werden kann und anschließend wieder in das Portgehäuse 102 einsetzbar ist.

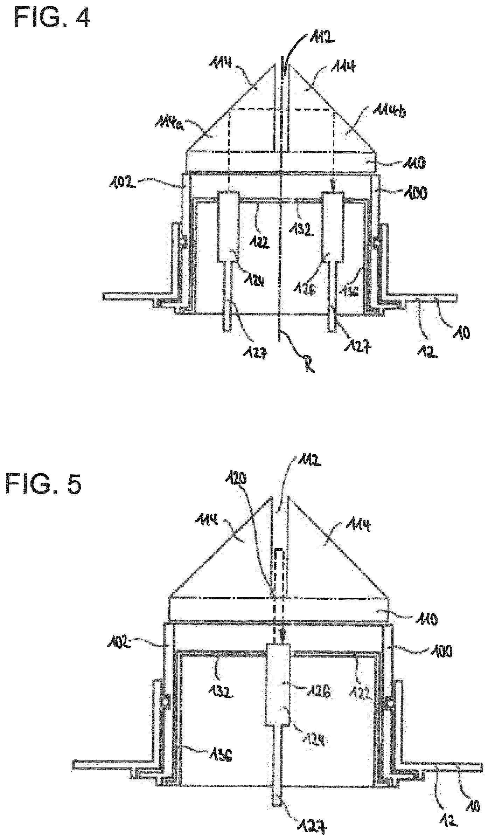

Der Messeinsatz aus Figur 1 eignet sich in vorteilhafter Weise zur Transmissionsmessung. Im Speziellen ist der Messeinsatz 122 derart ausgebildet, dass dieser zumindest ein Strahlung emittierendes Element 124 und zumindest ein Strahlung empfangendes Element 126 halten bzw. stützen kann. Figur 1b) zeigt eine Ausführungsform mit einem Messeinsatz 122, der ein Strahlung emittierendes Element 124 und ein Strahlung empfangendes Element 126 hält. Das Strahlung emittierende Element 124 ist dazu ausgelegt, elektromagnetische Strahlung auszusenden, während das Strahlung empfangende Element 126 dazu ausgelegt ist, elektromagnetische Strahlung zu empfangen. Das Strahlung emittierende Element 124 weist vorzugsweise zumindest einen Lichtleiter 127 auf. Zumindest ein Strahlungselement (hier nicht gezeigt), wie beispielsweise zumindest eine LED, kann elektromagnetische Strahlung über den zumindest einen Lichtleiter 127 über das Strahlung emittierende Element 124 ausgesendet werden. Vorzugsweise weist das Strahlung emittierende Element 124 an einem vorderen Ende ein Strahlungsauskopplungselement 128 auf. Über das Strahlungsauskopplungselement 128 kann die elektromagnetische Strahlung aus dem Strahlung emittierenden Element 124 austreten bzw. ausgekoppelt werden. Das Strahlung emittierende Element 124 ist dabei mit Hilfe des Messeinsatzes 122 vorzugsweise so angeordnet, dass die elektromagnetische Strahlung parallel zu der Rotationsachse R des Portgehäuses 102 ausgesendet bzw. ausgekoppelt werden kann und zu einem ersten Prisma 114a (erstes Umlenkungselement) gesendet werden kann. Die elektromagnetische Strahlung, welche auf das erste Prisma 114a trifft, wird durch das erste Prisma 114a derart umgelenkt, dass die elektromagnetische Strahlung durch den Messspalt 112 strahlt und zumindest teilweise auf das zweite Prisma 114b (zweites Umlenkungselement) trifft. Die elektromagnetische Strahlung wird im Messspalte 112 durch das in dem Messspalt 112 enthaltene Medium zumindest teilweise absorbiert und/oder reflektiert. Somit gelangt nur ein Anteil der ausgesendeten elektromagnetischen Strahlung zu dem zweiten Prisma 114b. Das zweite Prisma 114b wiederum lenkt die elektromagnetische Strahlung derart um, dass die elektromagnetische Strahlung zu dem Strahlung empfangenden Element 126 gesendet wird. Das Strahlung empfangende Element 126 umfasst vorzugsweise ein Strahlungseinkopplungselement 130, über das die empfangene Strahlung eingekoppelt werden kann. Zumindest ein Lichtleiter 127 leitet die empfangene Strahlung zu einem Photodetektor (hier nicht gezeigt), welcher dazu geeignet ist, die empfangene Strahlung zu empfangen und auszuwerten. Vorzugsweise ist das Strahlung empfangende Element 126 durch den Messeinsatz 122 derart in dem Portgehäuse 102 angeordnet, dass die elektromagnetische Strahlung parallel zu der Rotationsachse R des Portgehäuse 102 von dem zweiten Prisma 114b zu dem Strahlung empfangenden Element 126 strahlen kann. **Figur 4** zeigt Figur 1b) mit eingezeichnetem Verlauf der elektromagnetischen Strahlung.

Der Messeinsatz 122 weist zumindest eine Haltefläche 132 auf, welche im eingesetzten Zustand vorzugsweise senkrecht zu der Rotationsachse R des Portgehäuses 102 ausgerichtet ist. Die Haltefläche 132 weist Halteaussparungen 134 auf, in die jeweils ein Strahlung emittierendes Element 124 oder ein Strahlung empfangendes Element 126 einsetzbar ist. Die genannten Elemente 124, 126 können in die Halteeinsparung 134 einschraubbar sein oder durch eine Presspassung in der Halteeinsparung 134 gehalten werden. Wie in Figur 1b) gezeigt, wird vorzugsweise das Strahlungsauskopplungselement 128 und das Strahlungseinkopplungselement 130 von dem Messeinsatz 122 gehalten.

Weiterhin weist der Messeinsatz 122 zumindest eine Verbindungsfläche 136 auf. Die Verbindungsfläche 136 erstreckt sich vorzugsweise von der Haltefläche 132 zumindest bereichsweise parallel zu der Rotationsachse R des Portgehäuses 102 und ist im eingesetzten Zustand des Messeinsatzes 122 zumindest bereichsweise mit der Mantelfläche 106 des Portgehäuses 102 in Kontakt. Die Verbindungsfläche 136 ist lösbar mit der Mantelfläche 106 des Portgehäuses 102 verbindbar, so dass der Messeinsatz 122 während Sterilisationsverfahrens in einfacher Weise aus dem Portgehäuse 102 entnommen werden kann. Die Verbindungsfläche 136 kann beispielsweise zumindest einen Gummiring (hier nicht gezeigt) aufweisen, der eine stabile Verbindung zwischen dem Messeinsatz 122 und dem Portgehäuse 102 herstellt. Alternativ oder ergänzend kann der Messeinsatz in das Portgehäuse 102 einschraubbar sein. Ein hinteres Ende 138 der Verbindungsfläche 136 ist als freies Ende ausgebildet und kann, wie in Figur 1b) gezeigt, zur übrigen Verbindungsfläche 136 abgewinkelt ausgebildet sein. Vorzugsweise erstreckt sich das hintere Ende 138 der Verbindungsfläche 136 senkrecht zur Rotationsachse R des Portgehäuses 102. Dieses hintere Ende 138 kann dazu verwendet werden, dass der Messeinsatz nicht zu tief in das Portgehäuse 102 einsetzbar ist.

Die Verbindungsfläche 136 des Messeinsatzes 122 könnte im nicht eingesetzten Zustand auch derart ausgebildet sein, dass sich die Verbindungsfläche 136 von der Haltefläche 132 zu dem hinteren Ende 138 der Verbindungsfläche 136 aufweitet. Hierdurch bildet der Messeinsatz 122 eine Art Klammer, die durch ein Zusammenpressen des hinteren Endes 138 der Verbindungsflächen 136 ein Einsetzen des Messeinsatzes 122 in das Portgehäuse 102 ermöglicht. Der Messeinsatz 122 ist somit in das Portgehäuse 102 eingeklemmt.

Der Messeinsatz 122 ist vorzugsweise aus Metall oder Kunststoff gefertigt.

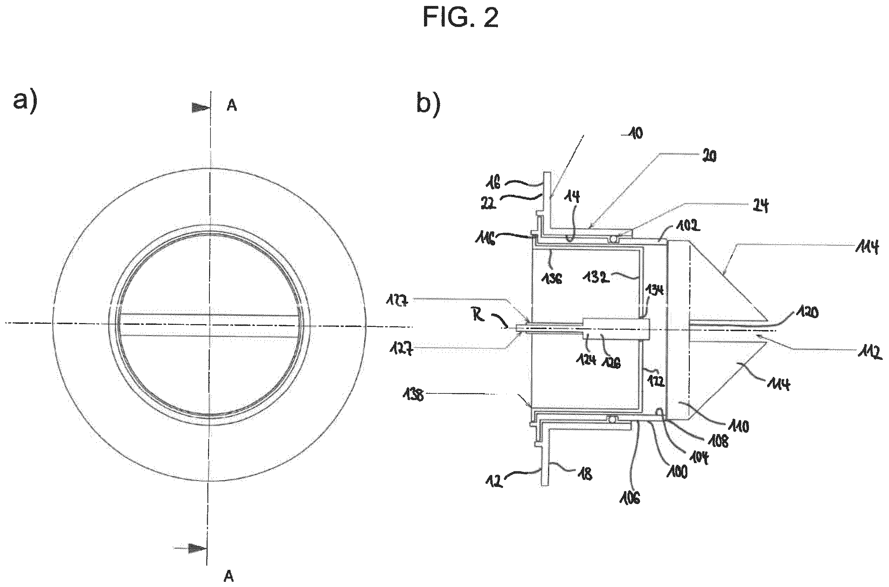

Die **Figuren 2a) und 2b****)** zeigen eine zweite Ausführungsform einer optischen Messvorrichtung 100. Es werden im Folgenden jedoch nur die Teile der optischen Messvorrichtung 100 beschrieben, die sich von der ersten Ausführungsform unterscheiden. Die Beschreibung der übrigen Teile der optischen Messvorrichtung 100 gilt entsprechend auch für die zweite Ausführungsform.

Die optische Messvorrichtung 100 aus den Figuren 2a) und 2b) eignet sich, im Gegensatz zu der optischen Messvorrichtung 100 aus den Figuren 1a) und 1b) zur Reflexionsmessung (z.B. Raman-Spektroskopie). Hierzu wird elektromagnetische Strahlung von dem Strahlung emittierenden Element 124 ausgesendet, vorzugsweise parallel zu der Rotationsachse R des Portgehäuses 102. Die elektromagnetische Strahlung wird anschließend von dem Medium, das sich in dem Bioprozessbehälter 10 befindet, reflektiert und/oder absorbiert. Gemessen wird jedoch nur der Anteil der elektromagnetischen Strahlung, der von dem Medium reflektiert wird. Hierzu befindet sich das Strahlung empfangende Element 126 vorzugsweise an derselben Stelle wie das Strahlung emittierende Element 124. Mit anderen Worten wird von dem Strahlung empfangenden Element 126 wiederum Strahlung empfangen, welche vorzugsweise parallel zu der Rotationsachse R des Portgehäuse 102 von dem Medium zu dem Strahlung empfangenden Element 126 reflektiert wird. Es ist dabei nicht zwingend erforderlich, dass die optische Messvorrichtung 100 einen Messspalt 112 aufweist. Wie in den Figuren 2a) und 2b) gezeigt, kann jedoch ein Messspalt 112 vorgesehen sein. Das Strahlung emittierende Element 124 und das Strahlung empfangende Element 126 sind dabei vorzugsweise im eingesetzten Zustand des Messeinsatzes 122 derart angeordnet, dass die elektromagnetische Strahlung durch die Bodenfläche 120 des Messspalts 112 strahlen kann. Es ist dabei entscheidend, dass die Bodenfläche 120 dabei zumindest bereichsweise als Fenster 110 ausgebildet ist. Weist die optische Messvorrichtung 100 keinen Messspalt 112 auf, können das Strahlung emittierende Element 124 und das Strahlung empfangende Element 126 im Vergleich zu Figur 2 auch parallel verschoben zur Rotationsachse R des Portgehäuses 102 angeordnet sein. Entscheidend ist hierbei lediglich, dass die genannten Elemente unterhalb eines Fensters 110 angeordnet sind. **Figur 5** zeigt Figur 2b) mit eingezeichnetem Verlauf der elektromagnetischen Strahlung.

Vorzugsweise ist das Strahlungsauskopplungselement 128 und das Strahlungseinkopplungselement 130 kombiniert ausgebildet, wie in Figur 2b) gezeigt. Die Lichtleiter 127 des Strahlung emittierenden Elements 124 und des Strahlung empfangenden Elements 126 sind vorzugsweise zu einem Faserbündel gebündelt.

Um das Strahlung empfangende Element 126, insbesondere zur Kalibrierung des Photodetektors, für die Reflexionsmessung kalibrieren zu können, kann parallel zur Rotationsrichtung R des Portgehäuses 102 bzw. seitlich versetzt zu dem Strahlung empfangenden Element 126 ein weiteres Strahlung emittierendes Element (hier nicht gezeigt) in dem Messeinsatz 122 angeordnet sein. In diesem Fall wird der Messspalt 112 durch zumindest ein Umlenkungselement begrenzt, unterhalb dem das weitere Strahlung emittierende Element angeordnet ist. Der Messspalt 112 kann auch hier beispielsweise durch zwei Umlenkungselemente ausgebildet sein, wie z.B. in Figur 1 gezeigt. Alternativ kann das zweite Umlenkungselement jedoch auch durch ein Begrenzungselement ohne Umlenkungsfunktion ersetzt werden.

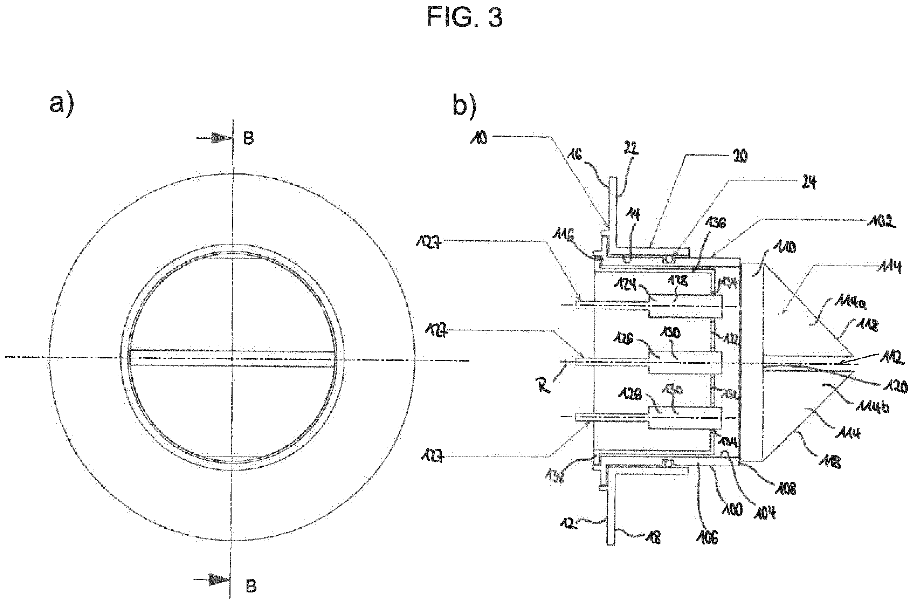

Die **Figuren 3a) und 3b****)** zeigen eine dritte Ausführungsform einer optischen Messvorrichtung 100. Hier werden mit Hilfe des Messeinsatzes 122 in einfacher Weise verschiedene Messverfahren miteinander in einer optischen Messvorrichtung 100 kombiniert werden.

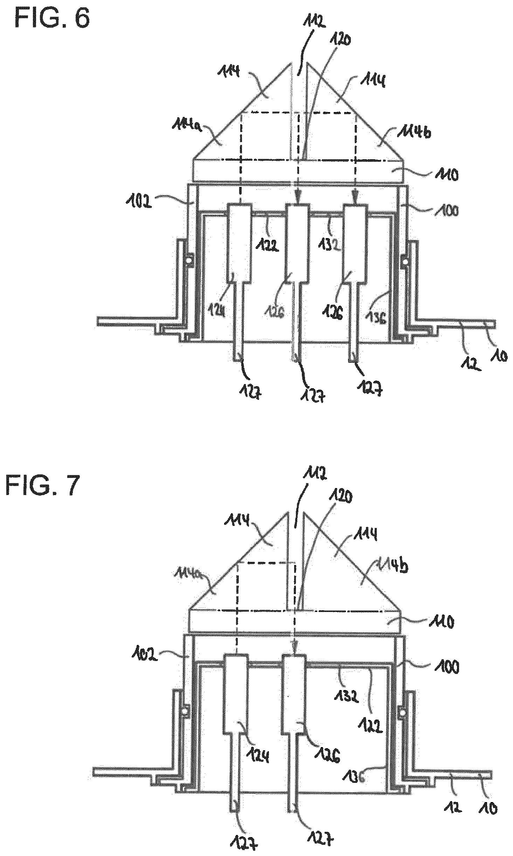

Konkret kann in der dritten Ausführungsform zum einen die bezüglich Figur 1 beschriebene Transmissionsmessung durchgeführt werden. Zusätzlich befindet sich hier jedoch unterhalb des Messspalts 112 eine weitere Strahlung empfangende Einheit 126, welche dazu ausgelegt ist, elektromagnetische Strahlung zu empfangen, die von dem Medium in dem Messspalt 112 umgelenkt bzw. abgelenkt bzw. nach Kontakt mit dem Medium abgestrahlt wird. Vorzugsweise handelt es sich hierbei um eine 90°-Detektion. **Figur 6** zeigt Figur 3b) mit eingezeichnetem Verlauf der elektromagnetischen Strahlung.

Bezüglich der Ausführungsform aus Figur 3 ist es weiterhin denkbar, dass nur unterhalb des Messspalts 112 zumindest ein Strahlung empfangendes Element 126 angeordnet ist. Das heißt, dass keine Transmissionsmessung durchgeführt wird, sondern lediglich der Anteil der elektromagnetischen Strahlung detektiert wird, der nach Kontakt mit dem Medium zu dem Strahlung empfangenden Element 126 unterhalb des Messspalts 112 abgestrahlt wird, gemessen wird. **Figur 7** zeigt eine Schnittansicht durch einen Teil eines Bioprozessbehälters 10 mit einer optischen Messvorrichtung 100, in der für eine 90°-Detektion der Verlauf der elektromagnetischen Strahlung eingezeichnet ist. Wie aus der Zeichnung ersichtlich ist, begrenzt hier das zweite Umlenkungselement lediglich des Messspalts 112. Eine Umlenkung der elektromagnetischen Strahlung erfolgt nicht.

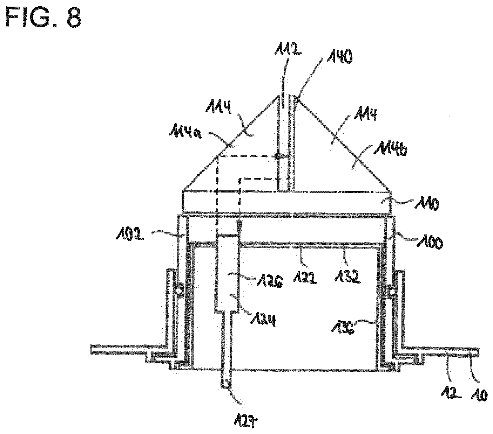

Weiterhin kann die in Figur 3 gezeigte Anordnung ebenfalls in vorteilhafter Weise für eine Transflexionsmessung genutzt werden. Hierzu ist die Fläche des zweiten Prismas 114b bzw. des zweiten Umlenkungselements, welche dem Messspalt 112 zugewandt ist, zumindest bereichsweise als diffus streuende Oberfläche 140 ausgebildet. Zumindest ein Teil der elektromagnetischen Strahlung, welche den Messspalt 112 passiert, wird an dieser diffus streuenden Oberfläche 140 reflektiert und kann anschließend von einem Strahlung empfangenden Element 126, welches sich unterhalb des ersten Prismas 114a befindet, empfangen bzw. detektiert werden. **Figur 8** zeigt eine Schnittansicht durch einen Teil eines Bioprozessbehälters 10 mit einer optischen Messvorrichtung 100, in der für eine Transflexionsmessung der Verlauf der elektromagnetischen Strahlung eingezeichnet ist.

Sowohl in der ersten als auch in der dritten Ausführungsform wird der Messspalt 112 durch die Prismen 114 gebildet, die mit dem Medium in dem Behältergehäuse 12 in Kontakt treten. Die Prismen 114 verbleiben während des Sterilisationsverfahrens im Bioprozessbehälter 10, während der Messeinsatz 122 aus dem Portgehäuse 102 entfernt wird. Es ist jedoch auch denkbar, dass die Prismen 114 oder die transparenten Elemente im Innenraum 104 des Portgehäuses 102 angeordnet sind und vorzugsweise mit dem Messeinsatz 122 verbunden sind. Somit können auch die Prismen 114 bzw. die transparenten Elemente während des Sterilisationsverfahrens aus dem Portgehäuse 102 entfernt werden. Die Prismen 114 oder die transparenten Elemente könnten mit Hilfe von Abstandshaltern an dem Messeinsatz 122 befestigt sein.

Um einen Messspalt 112 auszubilden, könnte das vordere Ende 108 des Portgehäuses 102 einen Messspalt 112 aufweisen. Im Speziellen könnte das vordere Ende 108 des Portgehäuses 102 einen ersten Vorsprung und einen zweiten Vorsprung aufweisen, in den jeweils ein Prisma 114 oder ein transparentes Element hineinragt. Die Flächen der Vorsprünge, welche sich gegenüberliegen und den Messspalt 112 eingrenzen, sind dabei zumindest bereichsweise als Fenster 110 ausgebildet.

Obwohl die transparenten Elemente als auch die Prismen 114 in den gezeigten Ausführungsformen aus zwei Teilen bestehen, könnten diese Umlenkungselemente auch aus einem speziell gefertigten Stück sein, das beispielsweise eine lateral verschobene 180° Reflexion erlaubt. In diesem Fall könnte der Messeinsatz 122 direkt durch Strukturen in den Umlenkungselementen orientiert werden (z.B. durch Stifte oder Nuten).

In den oben genannten Ausführungsformen wird das Fenster 110 als transparentes Element beschrieben, das transparent für elektromagnetische Strahlung ist. Es ist jedoch auch denkbar, dass es sich bei dem Fenster 110 um eine komplexere Optik handelt, die sich beispielsweise für Mikroskopie eignet.

Ausführungsform 2 zeigt eine Ausführungsform, in der mehrere Messtechniken in einer optischen Messvorrichtung kombiniert sind. Es ist weiterhin möglich, dass über die Art der gewählten Lichtleiter 127 verschiedenen Messtechniken kombiniert werden können.

### Bezugszeichenliste

- 10: Bioprozessbehälter
- 12: Behältergehäuse
- 14: Behältergehäuseausschnitt
- 16: Außenseite des Behältergehäuses
- 18: Innenraum des Behältergehäuses
- 20: Behältergehäusevorsprung
- 22: Mantelfläche des Behältergehäuses
- 24: Dichtungsring

- 100: Optische Messvorrichtung
- 102: Portgehäuse
- 104: Innenraum des Portgehäuses
- 106: Mantelfläche des Portgehäuses
- 108: Vorderes Ende des Portgehäuses
- 110: Fenster
- 112: Messspalt
- 114: Prisma
- 114a: Erstes Prisma
- 114b: Zweites Prisma
- 116: Hinteres Ende des Portgehäuses
- 118: Schräge Fläche
- 120: Bodenfläche des Messspalts
- 122: Messeinsatz
- 124: Strahlung emittierendes Element
- 126: Strahlung empfangendes Element
- 127: Lichtleiter
- 128: Strahlungsauskopplungselement
- 130: Strahlungseinkopplungselement
- 132: Haltefläche
- 134: Halteaussparung
- 136: Verbindungsfläche
- 138: Hinteres Ende der Verbindungsfläche
- 140: Diffus streuende Oberfläche

- R: Rotationsachse des Portgehäuses

## Patentansprüche

1. Bioprozessbehälter (10) mit einer optischen Messvorrichtung (100) zur nicht-invasiven spektroskopischen Messung umfassend:
• ein Behältergehäuse (12), welches geeignet ist, zumindest ein zu messendes Fluid aufzunehmen;
• ein Portgehäuse (102), welches mit dem Behältergehäuse (12) verbunden ist und gegenüber dem Innenraum (18) des Behältergehäuses (12) abgeschlossen ist;
• zumindest ein Strahlung emittierendes Element (124), welches dazu ausgelegt ist, elektromagnetische Strahlung durch das zumindest eine in dem Behältergehäuse (12) enthaltenen Fluid zu senden;
• zumindest ein Strahlung empfangendes Element (126), welches dazu ausgelegt ist, die Strahlung, welche von dem Strahlung emittierenden Element (124) ausgesendet wurde, zumindest teilweise zu empfangen; und
• zumindest einen Messeinsatz (122), welcher das zumindest eine Strahlung emittierende Element (124) und das zumindest eine Strahlung empfangende Element (126) hält bzw. stützt,
wobei der Messeinsatz (122) in das Portgehäuse (102) zumindest teilweise einschiebbar ist und im eingeschobenen Zustand mit dem Portgehäuse (102) lösbar verbunden ist;
wobei der Messeinsatz (122) Halteaussparungen (134) aufweist, in die das Strahlung emittierende Element (124) und das Strahlung empfangende Element (126) einsetzbar sind und
wobei der Messeinsatz (122) umfasst:
• zumindest eine Haltefläche (132), in der die Halteaussparungen (134) ausgebildet sind und welche im in das Portgehäuse (102) eingesetzten Zustand einem Fenster (110), einem ersten Umlenkelement und/oder zweiten Umlenkelement gegenüber angeordnet ist, und
• zumindest eine Verbindungsfläche (136), welche mit der zumindest einen Haltefläche (132) verbunden ist und dazu ausgelegt ist, mit dem Portgehäuse (102) im eingesetzten Zustand verbunden zu sein.

2. Bioprozessbehälter (10) nach Anspruch 1, wobei das Portgehäuse (102) zumindest ein Fenster (110) umfasst, welches ausgelegt ist, elektromagnetische Strahlung zwischen dem Innenraum (18) des Behältergehäuses (12) und einem Innenraum (104) des Portgehäuses (102) zumindest teilweise passieren zu lassen.

3. Bioprozessbehälter (10) nach Anspruch 1 oder 2, wobei das Portgehäuse (102) zumindest einen Messspalt (112) bzw. eine Messvertiefung aufweist, in den bzw. in die das zu messende Fluid aus dem Innenraum (18) des Behältergehäuses (12) einströmbar ist.

4. Bioprozessbehälter (10) nach Anspruch 3, wobei der Messspalt (112) zumindest zwei sich gegenüberliegende Fenster (110) aufweist, die derart voneinander beabstandet sind, dass zwischen den Fenstern (110) der Messspalt (112) ausgebildet ist.

5. Bioprozessbehälter (10) nach Anspruch 3, wobei der Messspalt (112) durch eine Strahlungsumlenkungsvorrichtung ausgebildet ist,
wobei die Strahlungsumlenkungsvorrichtung zumindest ein erstes Umlenkungselement und zumindest ein zweites Umlenkungselement aufweist,
wobei das erste und zweite Umlenkungselement einander gegenüber angeordnet sind und zumindest bereichsweise voneinander beabstandet sind, so dass der Messspalt (112) zwischen dem ersten und zweiten Umlenkungselement ausgebildet ist.

6. Bioprozessbehälter (10) nach Anspruch 4, wobei eine Strahlungsumlenkungsvorrichtung mit dem Messeinsatz (122) verbunden ist und zusammen mit dem Messeinsatz (122) in das Portgehäuse (102) einschiebbar ist,
wobei die Strahlungsumlenkungsvorrichtung zumindest ein erstes Umlenkungselement und zumindest ein zweites Umlenkungselement aufweist,
wobei das erste und zweite Umlenkungselement derart angeordnet sind, dass diese durch die zumindest zwei Fenster (110) und den Messspalt (112) beabstandet sind.

7. Bioprozessbehälter (10) nach Anspruch 5 oder 6, wobei das erste Umlenkungselement ausgelegt ist, elektromagnetische Strahlung, welche von dem Strahlung emittierenden Element (124) ausgesendet wird, zu empfangen und anschließend an die zweite Umlenkungseinheit derart umzulenken, dass die Strahlung den Messspalt (112) passiert, und
wobei die zweite Umlenkungseinheit ausgelegt ist, die Strahlung von der ersten Umlenkungseinheit zu empfangen und im Anschluss an das Strahlung empfangende Element (126) umzulenken.

8. Bioprozessbehälter (10) nach einem der Ansprüche 5 bis 7, wobei das erste Umlenkungselement ausgelegt ist, elektromagnetische Strahlung, welche von dem Strahlung emittierenden Element (124) ausgesendet wird, zu empfangen und anschließend zu dem Messspalt (112) umzulenken, und
wobei ein Strahlung empfangendes Element (126) unterhalb des Messspalts (112) angeordnet ist.

9. Bioprozessbehälter(10) nach einem der Ansprüche 1 bis 6, wobei das Strahlung emittierende Element (124) und das Strahlung empfangende Element (126) an derselben Position unterhalb des Messspalts (112) angeordnet sind.

10. Bioprozessbehälter (10) nach Anspruch 5 oder 6, wobei eine Fläche (140) des zweiten Umlenkungselements, welche dem Messspalt (112) zugewandt ist, zumindest bereichsweise diffus streuend ausgebildet ist,
wobei das erste Umlenkungselement ausgelegt ist, elektromagnetische Strahlung, welche von dem Strahlung emittierenden Element (124) ausgesendet wird, zu empfangen und anschließend zu dem Messspalt (112) und/oder der diffus streuenden Fläche (140) des zweiten Umlenkungselements umzulenken, und
wobei das Strahlung empfangende Element (126) derart angeordnet ist, dass dieses die elektromagnetische Strahlung, welche von dem zu messenden Fluid und/oder der diffus streuenden Fläche (140) reflektiert wird, messen kann.

11. Bioprozessbehälter (10) nach einem der Ansprüche 5 bis 10, wobei das erste und zweite Umlenkungselement jeweils ein Prisma (114) ist oder jeweils eine Strahlung reflektierende Fläche aufweist.

12. Bioprozessbehälter (10) nach einem der Ansprüche 5 oder 7 bis 11, wobei das erste und zweite Umlenkungselement auf zumindest einem Fenster (110) angeordnet sind.

13. Bioprozessbehälter (10) nach einem der vorangehenden Ansprüche, wobei der Messeinsatz (122) in das Portgehäuse (102) einklemmbar, mit dem Portgehäuse (102) verrastbar und/oder verschraubbar ist.

14. Bioprozessbehälter (10) nach einem der vorangehenden Ansprüche, wobei der Bioprozessbehälter ein Bioreaktor ist; und/oderwobei das Portgehäuse (102) zumindest teilweise in den Innenraum (18) des Behältergehäuses (12) ragt.

15. Optische Messvorrichtung (100) zur nicht-invasiven spektroskopischen Messung für einen Bioprozessbehälter (10) umfassend:
• ein Portgehäuse (102), welches mit einem Behältergehäuse (12) des Bioprozessbehälters (10) verbindbar ist und derart ausgebildet ist, dass dieses im verbundenen Zustand gegenüber dem Innenraum (18) des Behältergehäuses (12) abgeschlossen ist;
• zumindest ein Strahlung emittierendes Element (124), welches dazu ausgelegt ist, elektromagnetische Strahlung durch zumindest ein in dem Behältergehäuse (12) enthaltenes Fluid zu senden;
• zumindest ein Strahlung empfangendes Element (126), welches dazu ausgelegt ist, die Strahlung, welche von dem Strahlung emittierenden Element (124) ausgesendet wurde, zumindest teilweise zu empfangen; und
• zumindest einen Messeinsatz (122), welcher das zumindest eine Strahlung emittierende Element (124) und das zumindest eine Strahlung empfangende Element (126) hält bzw. stützt,
wobei der Messeinsatz (122) in das Portgehäuse (102) zumindest teilweise einschiebbar ist und im eingeschobenen Zustand mit dem Portgehäuse (102) lösbar verbunden ist;
wobei der Messeinsatz (122) Halteaussparungen (134) aufweist, in die das Strahlung emittierende Element (124) und das Strahlung empfangende Element (126) einsetzbar sind und
wobei der Messeinsatz (122) umfasst:
• zumindest eine Haltefläche (132), in der die Halteaussparungen (134) ausgebildet sind und welche im in das Portgehäuse (102) eingesetzten Zustand einem Fenster (110), einem ersten Umlenkelement und/oder zweiten Umlenkelement gegenüber anordenbar ist, und
• zumindest eine Verbindungsfläche (136), welche mit der zumindest einen Haltefläche (132) verbunden ist und dazu ausgelegt ist, mit dem Portgehäuse (102) im eingesetzten Zustand verbunden zu sein.

## Claims

1. Bioprocess container (10) with an optical measurement device (100) for non-invasive spectroscopic measurement comprising:
• a container housing (12), which is suitable for receiving at least one fluid to be measured;
• a port housing (102), which is connected to the container housing (12) and is closed off from the interior space (18) of the container housing (12);
• at least one radiation emitting element (124), which is designed to send electromagnetic radiation through at least one fluid contained in the container housing (12);
• at least one radiation receiving element (126), which is designed to at least partially receive the radiation which was emitted by the radiation emitting element (124); and
• at least one measurement insert (122), which supports and/or holds at least one radiation emitting element (124) and at least one radiation receiving element (126),
wherein the measurement insert (122) is at least partially insertable into the port housing (102) and is releasably connected to the port housing (102) in the inserted state;
wherein the measurement insert (122) has retaining recesses (134), into which the radiation emitting element (124) and the radiation receiving element (126) are insertable, and wherein the measurement insert (122) comprises:
• at least one retaining surface (132), in which the retaining recesses (134) are formed and which, in the state inserted into the port housing (102), is arranged opposite a window (110), a first deflecting element and/or a second deflecting element, and
• at least one connecting surface (136), which is connected to at least one retaining surface (132) and is designed to be connected to the port housing (102) in the inserted state.

2. Bioprocess container (10) according to claim 1, wherein the port housing (102) comprises at least one window (110), which is designed to at least partially allow electromagnetic radiation to pass between the interior space (18) of the container housing (12) and an interior space (104) of the port housing (102).

3. Bioprocess container (10) according to claim 1 or 2, wherein the port housing (102) has at least one measurement gap (112) or respectively a measurement recess, into which or respectively into which the fluid to be measured flows from the interior space (18) of the container housing (12).

4. Bioprocess container (10) according to claim 3, wherein the measurement gap (112) has at least two opposing windows (110), which are spaced apart from one another such that the measurement gap (112) is formed between the windows (110).

5. Bioprocess container (10) according to claim 3, wherein the measurement gap (112) is formed by a radiation deflecting device,
wherein the radiation deflecting device comprises at least a first deflecting element and at least a second deflecting element,
wherein the first and the second deflecting element are arranged opposite one another and are at least partially spaced apart from one another, so that the measurement gap (112) is formed between the first and the second deflecting element.

6. Bioprocess container (10) according to claim 4, wherein a radiation deflecting device is connected to the measurement insert (122) and is insertable together with the measurement insert (122) into the port housing (102),
wherein the radiation deflecting device comprises at least a first deflecting element and at least a second deflecting element,
wherein the first and the second deflecting element are arranged such that they, through at least two windows (110), delimit the measurement gap (112) being spaced apart from one another.

7. Bioprocess container (10) according to claim 5 or 6, wherein the first deflecting element is designed to receive electromagnetic radiation, which is emitted by the radiation emitting element (124), and to subsequently deflect it to the second deflecting unit such that the radiation passes through the measurement gap (112), and
wherein the second deflecting unit is designed to receive the radiation from the first deflecting unit and to deflect it to the radiation receiving element (126).

8. Bioprocess container (10) according to one of claims 5 to 7, wherein the first deflecting element is designed to receive electromagnetic radiation, which is emitted by the radiation emitting element (124), and to subsequently deflect it to the measurement gap (112), and
wherein a radiation receiving element (126) is arranged below the measurement gap (112).

9. Bioprocess container (10) according to one of claims 1 to 6, wherein the radiation emitting element (124) and the radiation receiving element (126) are arranged at the same position below the measurement gap (112).

10. Bioprocess container (10) according to claim 5 or 6, wherein a surface (140) of the second deflecting element, which faces the measurement gap (112), is formed at least partially as a diffusely scattering surface,
wherein the first deflecting element is designed to receive electromagnetic radiation, which is emitted by the radiation emitting element (124), and to deflect it to the measurement gap (112) and/or to the diffusely scattering surface (140) of the second deflecting element, and
wherein the radiation receiving element (126) is arranged such that it can measure the electromagnetic radiation, which is reflected by the fluid to be measured and/or by the diffusely scattering surface (140).

11. Bioprocess container (10) according to one of claims 5 to 10, wherein the first and the second deflecting element are each a prism (114) or each have a radiation reflecting surface.

12. Bioprocess container (10) according to one of claims 5 or 7 to 11, wherein the first and the second deflecting element are arranged on at least one window (110).

13. Bioprocess container (10) according to one of the preceding claims, wherein the measurement insert (122) is clampable into the port housing (102), engageable with the port housing (102) and/or screwable.

14. Bioprocess container (10) according to one of the preceding claims, wherein the bioprocess container is a bioreactor; and/or wherein the port housing (102) at least partially protrudes into the interior space (18) of the container housing (12).

15. Optical measurement device (100) for non-invasive spectroscopic measurement for a bioprocess container (10) comprising:
• a port housing (102), which is connectable to a container housing (12) of the bioprocess container (10) and is formed such that it, in the connected state, is closed off from the interior space (18) of the container housing (12);
• at least one radiation emitting element (124), which is designed to send electromagnetic radiation through at least one fluid contained in the container housing (12);
• at least one radiation receiving element (126), which is designed to at least partially receive the radiation which was emitted by the radiation emitting element (124); and
• at least one measurement insert (122), which supports and/or holds at least one radiation emitting element (124) and at least one radiation receiving element (126),
wherein the measurement insert (122) is at least partially insertable into the port housing (102) and is releasably connected to the port housing (102) in the inserted state;
wherein the measurement insert (122) has retaining recesses (134), into which the radiation emitting element (124) and the radiation receiving element (126) are insertable, and
wherein the measurement insert (122) comprises:
• at least one retaining surface (132), in which the retaining recesses (134) are formed and which, in the state inserted into the port housing (102), is arranged opposite a window (110), a first deflecting element and/or a second deflecting element, and
• at least one connecting surface (136), which is connected to at least one retaining surface (132) and is designed to be connected to the port housing (102) in the inserted state.

## Revendications

1. Récipient de bioprocédé (10) comportant un dispositif de mesure optique (100) pour une mesure spectroscopique non invasive comprenant :
• un boîtier de récipient (12), lequel est adapté pour recevoir au moins un fluide à mesurer ;
• un boîtier de port (102), lequel est relié au boîtier de récipient (12) et est fermé vis-à-vis de l'espace intérieur (18) du boîtier de récipient (12) ;
• au moins un élément émetteur de rayonnement (124), lequel est conçu pour envoyer un rayonnement électromagnétique à travers au moins un fluide contenu dans le boîtier de récipient (12) ;
• au moins un élément récepteur de rayonnement (126), lequel est conçu pour recevoir au moins partiellement le rayonnement qui a été émis par l'élément émetteur de rayonnement (124) ; et
• au moins un insert de mesure (122), lequel supporte et/ou maintient au moins un élément émetteur de rayonnement (124) et au moins un élément récepteur de rayonnement (126),
dans lequel l'insert de mesure (122) est au moins partiellement insérable dans le boîtier de port (102) et est relié de manière amovible au boîtier de port (102) à l'état inséré ;
dans lequel l'insert de mesure (122) présente des évidements de retenue (134), dans lesquels l'élément émetteur de rayonnement (124) et l'élément récepteur de rayonnement (126) sont insérables, et
dans lequel l'insert de mesure (122) comprend :
• au moins une surface de retenue (132), dans laquelle les évidements de retenue (134) sont formés et laquelle, dans l'état inséré dans le boîtier de port (102), est disposée en regard d'une fenêtre (110), d'un premier élément de déviation et/ou d'un second élément de déviation, et
• au moins une surface de liaison (136), laquelle est reliée à au moins une surface de retenue (132) et est conçue pour être reliée au boîtier de port (102) à l'état inséré.

2. Récipient de bioprocédé (10) selon la revendication 1, dans lequel le boîtier de port (102) comprend au moins une fenêtre (110), laquelle est conçue pour laisser passer au moins partiellement un rayonnement électromagnétique entre l'espace intérieur (18) du boîtier de récipient (12) et un espace intérieur (104) du boîtier de port (102).

3. Récipient de bioprocédé (10) selon la revendication 1 ou 2, dans lequel le boîtier de port (102) présente au moins une fente de mesure (112) ou respectivement un évidement de mesure, dans lequel ou respectivement dans laquelle le fluide à mesurer s'écoule depuis l'espace intérieur (18) du boîtier de récipient (12).

4. Récipient de bioprocédé (10) selon la revendication 3, dans lequel la fente de mesure (112) présente au moins deux fenêtres (110) situées en regard l'une de l'autre, lesquelles sont espacées l'une de l'autre de telle sorte que la fente de mesure (112) est formée entre les fenêtres (110).

5. Récipient de bioprocédé (10) selon la revendication 3, dans lequel la fente de mesure (112) est formée par un dispositif de déviation de rayonnement,
dans lequel le dispositif de déviation de rayonnement comprend au moins un premier élément de déviation et au moins un second élément de déviation,
dans lequel le premier et le second élément de déviation sont disposés en regard l'un de l'autre et sont espacés au moins partiellement l'un de l'autre, de sorte que la fente de mesure (112) est formée entre le premier et le second élément de déviation.

6. Récipient de bioprocédé (10) selon la revendication 4, dans lequel un dispositif de déviation de rayonnement est relié à l'insert de mesure (122) et est insérable conjointement avec l'insert de mesure (122) dans le boîtier de port (102),
dans lequel le dispositif de déviation de rayonnement comprend au moins un premier élément de déviation et au moins un second élément de déviation,
dans lequel le premier et le second élément de déviation sont disposés de telle sorte que ceux-ci, par au moins deux fenêtres (110), délimitent la fente de mesure (112) en étant espacés l'un de l'autre.

7. Récipient de bioprocédé (10) selon la revendication 5 ou 6, dans lequel le premier élément de déviation est conçu pour recevoir un rayonnement électromagnétique, lequel est émis par l'élément émetteur de rayonnement (124), et pour le dévier ensuite vers la seconde unité de déviation de telle sorte que le rayonnement passe à travers la fente de mesure (112), et
dans lequel la seconde unité de déviation est conçue pour recevoir le rayonnement provenant de la première unité de déviation et pour le dévier vers l'élément récepteur de rayonnement (126).

8. Récipient de bioprocédé (10) selon l'une des revendications 5 à 7, dans lequel le premier élément de déviation est conçu pour recevoir un rayonnement électromagnétique, lequel est émis par l'élément émetteur de rayonnement (124), et pour le dévier ensuite vers la fente de mesure (112), et
dans lequel un élément récepteur de rayonnement (126) est disposé en dessous de la fente de mesure (112).

9. Récipient de bioprocédé (10) selon l'une des revendications 1 à 6, dans lequel l'élément émetteur de rayonnement (124) et l'élément récepteur de rayonnement (126) sont disposés à la même position en dessous de la fente de mesure (112).

10. Récipient de bioprocédé (10) selon la revendication 5 ou 6, dans lequel une surface (140) du second élément de déviation, laquelle est tournée vers la fente de mesure (112), est formée au moins partiellement de manière diffusante,
dans lequel le premier élément de déviation est conçu pour recevoir un rayonnement électromagnétique, lequel est émis par l'élément émetteur de rayonnement (124), et pour le dévier vers la fente de mesure (112) et/ou vers la surface diffusante (140) du second élément de déviation, et
dans lequel l'élément récepteur de rayonnement (126) est disposé de telle sorte que celui-ci peut mesurer le rayonnement électromagnétique, lequel est réfléchi par le fluide à mesurer et/ou par la surface diffusante (140).

11. Récipient de bioprocédé (10) selon l'une des revendications 5 à 10, dans lequel le premier et le second élément de déviation sont chacun un prisme (114) ou présentent chacun une surface réfléchissant le rayonnement.

12. Récipient de bioprocédé (10) selon l'une des revendications 5 ou 7 à 11, dans lequel le premier et le second élément de déviation sont disposés sur au moins une fenêtre (110).

13. Récipient de bioprocédé (10) selon l'une des revendications précédentes, dans lequel l'insert de mesure (122) est insérable par encliquetage dans le boîtier de port (102), encliquetable avec le boîtier de port (102) et/ou vissable.

14. Récipient de bioprocédé (10) selon l'une des revendications précédentes, dans lequel le récipient de bioprocédé est un bioréacteur ; et/ou dans lequel le boîtier de port (102) fait au moins partiellement saillie dans l'espace intérieur (18) du boîtier de récipient (12).

15. Dispositif de mesure optique (100) pour une mesure spectroscopique non invasive pour un récipient de bioprocédé (10) comprenant :
• un boîtier de port (102), lequel est connectable à un boîtier de récipient (12) du récipient de bioprocédé (10) et est formé de telle sorte que celui-ci, à l'état connecté, est fermé vis-à-vis de l'espace intérieur (18) du boîtier de récipient (12) ;
• au moins un élément émetteur de rayonnement (124), lequel est conçu pour envoyer un rayonnement électromagnétique à travers au moins un fluide contenu dans le boîtier de récipient (12) ;
• au moins un élément récepteur de rayonnement (126), lequel est conçu pour recevoir au moins partiellement le rayonnement qui a été émis par l'élément émetteur de rayonnement (124) ; et
• au moins un insert de mesure (122), lequel supporte et/ou maintient au moins un élément émetteur de rayonnement (124) et au moins un élément récepteur de rayonnement (126),
dans lequel l'insert de mesure (122) est au moins partiellement insérable dans le boîtier de port (102) et est relié de manière amovible au boîtier de port (102) à l'état inséré ;
dans lequel l'insert de mesure (122) présente des évidements de retenue (134), dans lesquels l'élément émetteur de rayonnement (124) et l'élément récepteur de rayonnement (126) sont insérables, et
dans lequel l'insert de mesure (122) comprend :
• au moins une surface de retenue (132), dans laquelle les évidements de retenue (134) sont formés et laquelle, dans l'état inséré dans le boîtier de port (102), est disposée en regard d'une fenêtre (110), d'un premier élément de déviation et/ou d'un second élément de déviation, et
• au moins une surface de liaison (136), laquelle est reliée à au moins une surface de retenue (132) et est conçue pour être reliée au boîtier de port (102) à l'état inséré.
